# EUROPÄISCHE PATENTSCHRIFT

(11) **EP 2 481 348 B1**
(45) Veröffentlichungstag und Bekanntmachung des Hinweises auf die Patenterteilung: **21.12.2016**
(21) Anmeldenummer: 12161995.1
(22) Anmeldetag: 09.10.2004
(51) Int. Cl.: A61B 5/00

(54) **Handgerät zur Untersuchung einer Körperflüssigkeit**
Handheld device for testing a bodily fluid
Appareil manuel destiné à l'examen d'un échantillon de liquide corporel

(30) Priorität: 17.10.2003 DE 10348283
(43) Veröffentlichungstag der Anmeldung: 01.08.2012
(62) Teilanmeldung aus: 04790236.6
(73) Patentinhaber: F. Hoffmann-La Roche AG, 4070 Basel (CH); Roche Diabetes Care GmbH, 68305 Mannheim (DE)
(72) Erfinder: Harttig, Herbert, Dr., 67434 Neustadt (DE); List, Hans, 64754 Hesseneck-Kailbach (DE)
(74) Vertreter: Pfiz, Thomas

(56) Entgegenhaltungen:
- WO-A-01/23885
- WO-A-02/100274
- DE-A1- 19 819 407
- DE-A1- 19 849 539
- US-A- 5 228 972

## Beschreibung

Die Erfindung betrifft ein Handgerät zur Untersuchung einer Körperflüssigkeit, insbesondere ein tragbares Blutzuckermessgerät, mit einer Bandeinheit zum abschnittsweisen Vorspulen eines Testbandes, einer Detektionseinheit zur Messwerterfassung an den im Bereich einer Aufnahmestelle mit Körperflüssigkeit beaufschlagten Testbandabschnitten und einem Gehäuse zur Aufnahme der Bandeinheit und Detektionseinheit.

Für Diabetiker sind regelmäßige Blutzuckerkontrollen unerlässlich, um Therapie, Ernährung und Lebensrhythmus auf die jeweiligen Erfordernisse einstellen zu können. Zur Selbstkontrolle sind gleichsam als Minilabore arbeitende Handgeräte am Markt, mit denen auch von Laien die erforderlichen Schritte einfach und schnell vorgenommen werden können. Dabei werden disposible Teststreifen bereitgehalten, um nach Beaufschlagung mit Kapillarblut einen geräteinternen Nachweis beispielsweise durch eine optische Messeinheit zu ermöglichen. Die Magazinierung und Verarbeitung der Teststreifen beansprucht allerdings viel Bauraum und macht aufwendige Antriebe erforderlich. In den Patentschriften US5228972 und EP1424040 wird deshalb vorgeschlagen, anstelle einzelner Teststreifen ein aufgewickeltes Testband zu verwenden, auf dem eine Vielzahl von mit einer geeigneten Testchemie versehenen Testabschnitten fortlaufend angeordnet sind. Die Körperflüssigkeit wird auf einem durch Bandvorlauf in eine aktive Position gebrachten Testabschnitt appliziert und analysiert. Einzelheiten zur Blutgewinnung sowie zu bekannten Testmedien und Nachweissystemen insbesondere für Blutglucose sind dort angegeben, worauf hier Bezug genommen wird. Für eine genaue Dosierung möglichst geringer Blutmengen wurde bereits vorgeschlagen, das Testband über eine gehäuseexterne Umlenkspitze zu exponieren. Problematisch dabei ist der Schutz der aufwändigen empfindlichen Messanordnung gegen äußere Einflüsse und die exakte Ausrichtung der Nachweisoptik.

Ausgehend hiervon liegt der Erfindung die Aufgabe zugrunde, die im Stand der Technik aufgetretenen Nachteile zu vermeiden und ein robustes und zuverlässig arbeitendes Gerät zu schaffen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Demnach wird erfindungsgemäß vorgeschlagen, dass die Bandeinheit und/oder die Detektionseinheit bei einer Stoßbelastung gegen ein nachgiebiges Rückstellelement aus einer Arbeitsstellung gegenüber dem Gehäuse auslenkbar sind. Dadurch ist es möglich, dass die empfindliche Messanordnung im Falle einer äußeren Krafteinwirkung nicht übermäßig beansprucht wird, sondern in das Geräteinnere hinein ausweichen kann, so dass die Hauptbelastung von stabilen Gehäusestrukturen aufgenommen werden kann. Zudem kann durch die Rückstellkraft eine genaue Zentrierung in der Arbeitsstellung erreicht werden, ohne dass die betreffenden Strukturen mit äußerster Präzision gefertigt werden müssten.

Für einen besonders guten Schutz ist es vorteilhaft, wenn die Bandeinheit und/oder die Detektionseinheit von der Aufnahmestelle weg in das Gehäuse hinein auslenkbar sind.

Eine bevorzugte Ausführung sieht vor, dass die Detektionseinheit über das Rückstellelement an dem Gehäuse elastisch abgestützt ist.

Für die Krafteinleitung und Positionierung in der Arbeitsstellung ist es von Vorteil, wenn die Bandeinheit und die Detektionseinheit über mindestens einen starren Anschlag, insbesondere gegenseitige Eingriffsflächen unter der Rückstellkraft des Rückstellelements in definierter Lagebeziehung zueinander gehalten sind.

Zur Vereinfachung der Handhabung ist es günstig, wenn die Bandeinheit und die Detektionseinheit über einen auf das Rückstellelement einwirkenden Freigabemechanismus aus einer gegenseitigen Anschlaglage lösbar sind. Dabei ist der Freigabemechanismus vorteilhafterweise mit einem Gehäusedeckel gekoppelt, so dass das Auswechseln der Bandeinheit weiter vereinfacht wird.

Für eine begrenzte Bewegungsfreiheit bei der Stoßaufnahme ist es vorteilhaft, wenn die Detektionseinheit und/oder die Bandeinheit vorzugsweise mit großem, d.h. nicht durch Herstellungstoleranzen bedingtem Spiel in einer Gehäuseführung gelagert sind. Eine vorteilhafte Ausführung sieht vor, dass die Detektionseinheit und/oder die Bandeinheit über einen Schlitten in dem Gehäuse linearbeweglich geführt sind. Eine weitere Vereinfachung wird dadurch erreicht, dass der Schlitten über ein Getriebe mittels eines Vorspulantriebs für das Testband antreibbar ist.

Vorteilhafterweise ist das Rückstellelement elastisch, insbesondere federelastisch oder gummielastisch ausgebildet. Hierbei ist es bevorzugt, dass das Rückstellelement durch eine Feder, insbesondere eine in der Arbeitsstellung vorgespannte Druckfeder gebildet ist.

Erfindungsgemäss weist die Bandeinheit einen in der Arbeitsstellung aus dem Gehäuse herausragenden Aufnahmekopf zur gehäuseexternen Exposition des Testbandes auf. Zur initialen Lastaufnahme ist es günstig, wenn der vorzugsweise aus Federstahl bestehende Aufnahmekopf elastisch verformbar ausgebildet ist.

Ein weiterer besonderer Erfindungsaspekt besteht darin, dass die Aufnahmestelle durch eine Abdeckvorrichtung in einer Schließstellung gegen äußere Einwirkung abgeschirmt und in einer Öffnungsstellung zur Applikation von Körperflüssigkeit freigegeben ist. Dadurch ist es möglich, empfindliche Gerätestrukturen gegen äußere Kräfte und auch gegen das Eindringen von Verunreinigungen zu schützen. Dabei wirkt der Schutz im Ruhezustand, nicht aber im Moment der Benutzung.

Erfindungsgemäss weist die Abdeckvorrichtung ein als Schieber oder Rollo ausgebildetes, gegenüber dem Gehäuse bewegliches Verschlussteil auf. Dabei sollte die Abdeckvorrichtung zum Verschluss einer die Aufnahmestelle begrenzenden Gehäuseöffnung ausgebildet sein.

Eine weitere Verbesserung sieht vor, dass die Abdeckvorrichtung bei einer Geräteaktivierung selbsttätig in die Öffnungsstellung bringbar ist. Hierbei ist es günstig, wenn durch einen beim Vorspulen des Testbandes aktivierten Bandantrieb zugleich die Abdeckvorrichtung in die Öffnungsstellung bewegbar ist.

Um die Schutzwirkung weiter zu verbessern, ist es von Vorteil, wenn die Abdeckvorrichtung bei manueller Gerätehandhabung in die Öffnungsstellung und beim Loslassen des Gehäuses selbsttätig in die Schließstellung bringbar ist.

Zur Vereinfachung der Handhabung und der Schließfunktion ist es vorteilhaft, wenn die Abdeckvorrichtung einen vorzugsweise durch Betätigung von an dem Gehäuse befindlichen Griffelementen in der Öffnungsstellung vorgespannten Federmechanismus aufweist. Denkbar ist es auch, eine geräteinternen Stromversorgung beim Öffnen der Abdeckvorrichtung durch ein Schaltelement selbsttätig einzuschalten.

Vorteilhafterweise ist die Bandeinheit durch eine austauschbare Wechselkassette gebildet, während die Testbandabschnitte durch auf einem kontinuierlichen Trägerband abschnittsweise aufgebrachte, auf einen Inhaltsstoff der Körperflüssigkeit ansprechende Reagenzfelder gebildet sind.

Im folgenden wird die Erfindung anhand eines in der Zeichnung in schematischer Weise dargestellten Ausführungsbeispiels näher erläutert. Es zeigen
- Fig. 1: ein stoßgeschützes Blutzuckermessgerät für Diabetiker in teilweise geschnittener Breitseitenansicht;
- Fig. 2: das Blutzuckermessgerät in einem zu Fig. 1 senkrechten Längsschnitt;
- Fig. 3: eine weitere Ausführungsform eines Blutzuckermessgeräts mit einer Verschlussvorrichtung in einer Öffnungs- und Schließstellung;
- Fig. 4: das Blutzuckermessgerät bei geöffneter Verschlussvorrichtung zur Blutapplikation auf einem Testband in teilweise aufgebrochener Darstellung; und
- Fig. 5: das Blutzuckermessgerät bei geschlossener Verschlussvorrichtung in einer Fig. 4 entsprechenden Darstellung.

Das in der Zeichnung dargestellte Blutzuckermessgerät umfasst ein in der Hand aufnehmbares Gehäuse 10, eine darin eingesetzte Bandkassette 12 mit Testband 14, eine optisch arbeitende Detektionseinheit 16 zur Analyse von auf dem Testband appliziertem Blut sowie ein federelastisches Rückstellelement 18 und/oder eine Abdeck- bzw. Verschlussvorrichtung 20 als Aufprallschutz.

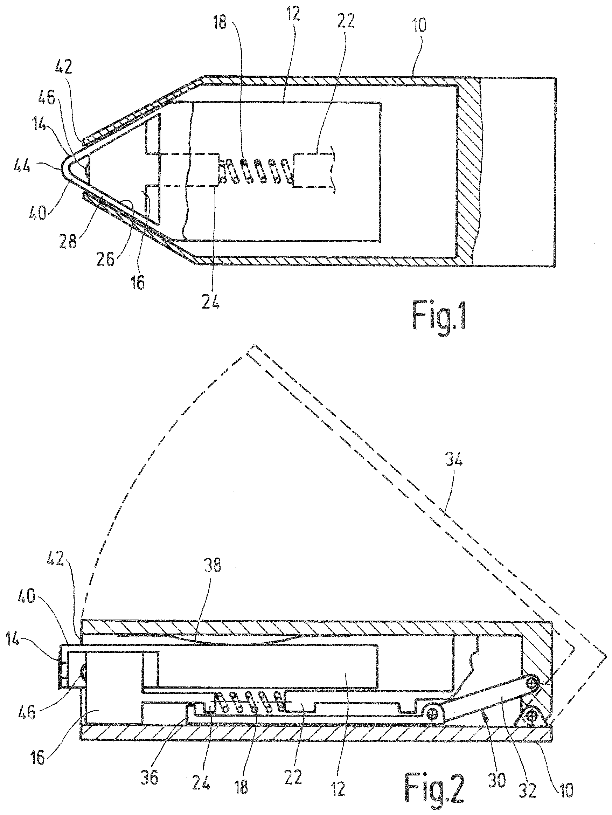

Wie in Fig. 1 dargestellt, ist das Rückstellelement 18 durch eine Schraubendruckfeder gebildet, die mit ihren Federenden zwischen einer gehäusefesten Abstützung 22 und einem Ausleger 24 der Detektionseinheit 16 in der Gerätelängsachse vorgespannt ist. Die Rückstellkraft der Schraubendruckfeder 18 wirkt dabei so, dass die Detektionseinheit 16 und die Bandeinheit 12 über keilförmige Anschlagflächen 26, 28 gegeneinander und gegen das Gehäuse 10 in definierter Anschlaglage gehalten sind.

Gemäß Fig. 2 ist die Bandkassette 12 zum Auswechseln durch einen Freigabemechanismus 30 aus ihrer Anschlaglage lösbar. Der Freigabemechanismus 30 weist einen Kopplungshebel 32 auf, der an seinen Enden an einer Gehäuseklappe 34 und an einem Zughaken 36 angelenkt ist. Beim Aufschwenken der Gehäuseklappe 34 (gestrichelt in Fig. 2) wird der Zughaken 36 zurückgezogen. Er greift dabei etwa auf halbem Rückzugweg in den Ausleger 24 der Detektionseinheit 16 ein und zieht diesen gegen die Kraft der Rückstellfeder zurück, so dass die Bandkassette 12 freikommt und entnommen werden kann. Bei geschlossener Gehäuseklappe 34 sorgt eine Blattfeder 38 dafür, dass die Bandkassette 12 auf der Detektionseinheit 16 und im Gehäuse 10 kraftschlüssig aufliegt.

In der in Fig. 1 und 2 gezeigten Anschlaglage befinden sich die Bandkassette 12 und die Detektionseinheit 16 in einer Arbeitsstellung zur Messwerterfassung. Hierbei ragt ein spitz zulaufender Aufnahmekopf 40 der Bandkassette 12 aus einer Messöffnung 42 des Gehäuses 10 heraus und ermöglicht so eine dosierte Blutapplikation an der Aufnahmestelle 44 im Bereich seiner Spitze. Der Aufnahmekopf 40 bildet dabei eine Führung für das nach außen exponierte Testband 14, welches im Inneren der Bandkassette 12 über nicht gezeigte Wickelspulen abschnittsweise vorgespult wird.

Das Testband 14 weist eine Vielzahl von auf einem kontinuierlichen transparenten Trägerband im Abstand voneinander aufgebrachte Reagenzfelder auf, die durch entsprechenden Bandvorlauf sukzessive zum Einsatz gebracht werden und auf einen Analyten in einem aufgebrachten Blutstropfen durch eine Farbänderung ansprechen. Der Nachweis dieser Reaktion erfolgt durch reflexionsphotometrische Messung mittels der Optik 46 der Detektionseinheit 16, die in der Arbeitsstellung exakt auf Aufnahmestelle 44 fokussiert ist.

Bei einer Stoßbelastung beispielsweise durch ungewolltes Fallenlassen des Geräts kann der Aufnahmekopf 40 in Kontakt mit der empfindlichen Detektionseinheit 16 gegen die Rückstellkraft der Schraubendruckfeder 18 in das Gehäuseinnere hinein ausweichen, so dass die Last im wesentlichen von dem stabilen Gehäuse 10 aufgenommen wird. Die Detektionseinheit 16 hat dabei gegenüber dem Zughaken 36 ausreichend Freiraum, um den Federweg nutzen zu können. Zudem besteht außerhalb der Anschlaglage in allen Freiheitsgraden ein begrenztes Bewegungsspiel bezüglich des Gehäuses, so dass auch ein nicht axial gerichteter Stoß günstig abgefangen werden kann. Zweckmäßig ist der Aufnahmekopf 40 beispielsweise als Federstahlführung elastisch verformbar ausgebildet, um anfängliche Kraftspitzen abzufedern.

Wie nicht eigens dargestellt, können die Detektionseinheit 16 und die Bandkassette 12 auf einem Schlitten in dem Gehäuse linearbeweglich geführt sein. Als Antrieb kann der ohnehin erforderliche Wickelantrieb für das Testband genutzt werden, wobei die Drehbewegung über ein Getriebe in eine Linearbewegung des Schlittens umgesetzt wird.

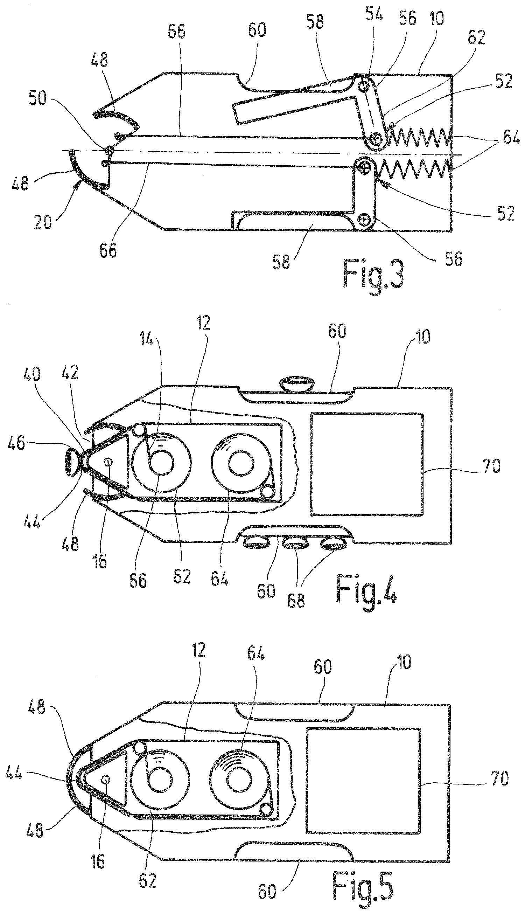

Gemäss Fig. 3 bis 5 ist der Bereich der Aufnahmestelle 44 in der Öffnungsstellung der Verschlussvorrichtung 20 zur Blutapplikation beispielsweise aus einer Fingerkuppe 46 freigegeben (Fig. 4) und in der Schließstellung gegen äußere Krafteinwirkung abgeschirmt (Fig. 5). Zu diesem Zweck weist die Verschlussvorrichtung 20 zwei halbschalige Verschlussklappen 48 auf, welche um eine gemeinsame Drehachse 52 verschwenkbar sind.

Gemäß Fig. 3 ist jede Verschlussklappe 48 über einen zugehörigen Federmechanismus 50 betätigbar. Dieser umfasst einen im Drehpunkt 54 am Gehäuse 10 angelenkten zweiarmigen Griffhebel 56, dessen Griffarm 58 in eine Griffmulde 60 des Gehäuses 10 ragt und dessen in das Geräteinnere abgewinkelter Stützarm 62 über eine Feder 64 am Gehäuse 10 abgestützt und über eine Stange 66 mit einer Verschlussklappe 48 verbunden ist. Unter der Federvorspannung wird die Verschlussklappe 48 in der Schließstellung gehalten, wie es in Fig. 3 für die untere Gehäusehälfte gezeigt ist. Wird der Griffarm 58 manuell entgegen der Kraft der Feder 64 betätigt, so zieht die Stange 66 die Verschlussklappe 48 in die in Fig. 3 oben gezeigte Öffnungsstellung auf. Auf diese Weise ist es möglich, dass die Schließstellung beim gewollten oder unbeabsichtigten Loslassen automatisch eingenommen wird.

Die in Fig. 4 und 5 gezeigte Ausführungsform sieht elektrische Griffschalter 60 vor, die in der federbelasteten gedrückten Schaltstellung das Gerät aktivieren und dabei eine motorische Öffnung der Verschlussklappen 48 bewirken. Hierfür kann ein die Wickelspulen 62, 64 der Bandkassette 12 antreibender Bandantrieb 66 genutzt werden, welcher über geeignete Getriebemittel mit den Verschlussklappen 48 koppelbar ist. Im aktivierten Zustand wird das Gerät mit den Fingern 68 in einer Hand gehalten, während an einem Finger 46 der anderen Hand wie vorstehend beschrieben Blut aufgenommen und analysiert wird, wobei das Messergebnis über eine Anzeige 70 ablesbar ist. Beim Ablegen des Geräts werden die Verschlussklappen 48 über der Öffnung 42 automatisch geschlossen und somit die Aufnahmestelle 44 gegen eindringende Verunreinigungen abgeschirmt.

## Patentansprüche

1. Handgerät zur Untersuchung einer Körperflüssigkeit, insbesondere tragbares Blutzuckermessgerät, mit einer durch eine austauschbare Wechselkassette gebildeten Bandeinheit (12) zum abschnittsweisen Vorspulen eines Testbandes (14), einer Detektionseinheit (16) zur Messwerterfassung an den im Bereich einer Aufnahmestelle (44) mit Körperflüssigkeit beaufschlagten Testbandabschnitten, und einem Gehäuse (10) zur Aufnahme der Bandeinheit (12) und Detektionseinheit (16), wobei die Aufnahmestelle (44) durch eine Abdeckvorrichtung (20) in einer Schließstellung gegen äußere Einwirkung abgeschirmt und in einer Öffnungsstellung zur Applikation von Körperflüssigkeit freigegeben ist, **dadurch gekennzeichnet, dass** die Abdeckvorrichtung (20) mindestens ein als Schieber oder Rollo ausgebildetes, gegenüber dem Gehäuse (10) bewegliches Verschlussteil (48) aufweist, und dass die Bandeinheit (12) einen in der Arbeitsstellung aus dem Gehäuse (10) herausragenden Aufnahmekopf (40) zur gehäuseexternen Exposition des Testbandes (14) aufweist.

2. Handgerät nach Anspruch 1, **dadurch gekennzeichnet, dass** die Abdeckvorrichtung (20) zum Verschluss einer die Aufnahmestelle (44) begrenzenden Gehäuseöffnung (42) ausgebildet ist.

3. Handgerät nach Anspruch 1 oder 2, **dadurch gekennzeichnet, dass** die Abdeckvorrichtung (20) bei einer Geräteaktivierung selbsttätig in die Öffnungsstellung bringbar ist.

4. Handgerät nach einem der Ansprüche 1 bis 3, **dadurch gekennzeichnet, dass** durch einen beim Vorspulen des Testbandes (14) aktivierten Bandantrieb (66) zugleich die Abdeckvorrichtung (20) in die Öffnungsstellung bewegbar ist.

5. Handgerät nach einem der Ansprüche 1 bis 4, **dadurch gekennzeichnet, dass** die Abdeckvorrichtung (20) bei manueller Gerätehandhabung in die Öffnungsstellung und beim Loslassen des Gehäuses (10) selbsttätig in die Schließstellung bringbar ist.

6. Handgerät nach einem der Ansprüche 1 bis 5, **dadurch gekennzeichnet, dass** die Abdeckvorrichtung (20) einen vorzugsweise durch Betätigung von an dem Gehäuse (10) befindlichen Griffelementen ((56) in der Öffnungsstellung vorgespannten Federmechanismus (52) aufweist.

7. Handgerät nach einem der Ansprüche 1 bis 6, **gekennzeichnet durch** ein Schaltelement zum selbsttätigen Einschalten einer geräteinternen Stromversorgung beim Öffnen der Abdeckvorrichtung (20).

8. Handgerät nach einem der Ansprüche 1 bis 7, **dadurch gekennzeichnet, dass** die Testbandabschnitte durch auf einem kontinuierlichen Trägerband abschnittsweise aufgebrachte, auf einen Inhaltsstoff der Körperflüssigkeit ansprechende Reagenzfelder gebildet sind.

9. Handgerät nach einem der Ansprüche 1 bis 8, **dadurch gekennzeichnet, dass** die Bandeinheit (12) und/oder die Detektionseinheit (16) bei einer Stoßbelastung gegen ein nachgiebiges Rückstellelement (18) aus einer Arbeitsstellung gegenüber dem Gehäuse (10) auslenkbar sind.

10. Handgerät nach Anspruch 9, **dadurch gekennzeichnet, dass** die Bandeinheit (12) und/oder die Detektionseinheit (16) von der Aufnahmestelle (44) weg in das Gehäuse (10) hinein auslenkbar sind.

11. Handgerät nach Anspruch 9 oder 10, **dadurch gekennzeichnet, dass** die Bandeinheit (12) und die Detektionseinheit (16) über einen auf das Rückstellelement (18) einwirkenden Freigabemechanismus (30) aus einer gegenseitigen Anschlaglage lösbar sind.

12. Handgerät nach Anspruch 11, **dadurch gekennzeichnet, dass** der vorzugsweise aus Federstahl bestehende Aufnahmekopf (40) elastisch verformbar ausgebildet ist.

## Claims

1. Handheld device for analysing a body fluid and in particular a portable blood sugar measuring device comprising a tape unit (12) formed as an exchangeable cassette to wind a test tape (14) forwards in sections, a detection unit (16) for detecting measured values on the sections of tape to which body fluid has been applied in the area of a receiving site (44), and a housing (10) to hold the tape unit (12) and detection unit (16), wherein in a closed position the receiving site (44) is screened from external effects by a cover device (20) and in an open position is released in order to apply body fluid, **characterized in that** the cover device (20) has at least one closing member (48) which can move relative to the housing (10) and is in the form of a slider or shutter and that the tape unit (12) has a receiving head (40) which protrudes from the housing (10) in the operating position in order to expose the test tape (14) outside the housing.

2. Handheld device according to claim 1, **characterized in that** the cover device (20) is designed to close an opening in the housing (42) which bounds the receiving site (44).

3. Handheld device according to claim 1 or 2, **characterized in that** when the device is activated, the cover device (20) can be automatically moved into the open position.

4. Handheld device according to one of the claims 1 to 3, **characterized in that** a tape drive (66) that is activated when forwarding the test tape (14) at the same time moves the cover device (20) into the open position.

5. Handheld device according to one of the claims 1 to 4, **characterized in that** the cover device (20) can be moved into the open position when the device is operated manually and can be automatically moved into the closed position when the housing (10) is released.

6. Handheld device according to one of the claims 1 to 5, **characterized in that** the cover device (20) has a spring mechanism (52) which is pretensioned in the open position preferably by actuating grip elements (56) located on the housing (10).

7. Handheld device according to one of the claims 1 to 6, **characterized by** a switching element for automatically switching on a power supply inside the device when the cover device (20) is opened.

8. Handheld device according to one of the claims 1 to 7, **characterized in that** the test tape sections are formed by reagent fields that react to a constituent of the body fluid and are applied in sections to a continuous carrier tape.

9. Handheld device according to one of the claims 1 to 8, **characterized in that** the tape unit (12) and/or the detection unit (16) can be deflected relative to the housing (10) from an operating position against a flexible restoring element (18) when subjected to a shock load.

10. Handheld device according to claim 9, **characterized in that** the tape unit (12) and/or the detection unit (16) can be deflected away from the receiving site (44) into the housing (10).

11. Handheld device according to claim 9 or 10, **characterized in that** the tape unit (12) and the detection unit (16) can be detached from a mutual stop position by means of a release mechanism (30) acting on the restoring element (18).

12. Handheld device according to claim 11, **characterized in that** the receiving head (40) which preferably consists of spring steel is designed to be elastically deformable.

## Revendications

1. Appareil à main destiné à l'examen d'un fluide corporel, en particulier appareil de mesure de glycémie portatif, avec une unité de bande (12), constituée d'une cassette interchangeable, destinée à un prébobinage par sections d'une bande de test (14), une unité de détection (16) destinée à une collecte de valeurs de mesure au niveau des sections de bande de test soumises au fluide corporel dans le secteur d'un point de réception (44), et un boîtier (10) destiné à recevoir l'unité de bande (12) et l'unité de détection (16), dans lequel le point de réception (44) est, dans une position de fermeture, protégé contre une action extérieure grâce à un dispositif d'obturation (20) et, dans une position d'ouverture, est ouvert en vue d'une application de fluide corporel, **caractérisé en ce que** le dispositif d'obturation (20) présente au moins une pièce de verrouillage (48) mobile par rapport au boîtier (10), réalisée sous forme de rideau ou de volet, et l'unité de bande (12) présente une tête de réception (40) faisant saillie hors du boîtier (10) dans la position de travail en vue d'une exposition, externe au boîtier, de la bande de test (14).

2. Appareil à main selon la revendication 1, **caractérisé en ce que** le dispositif d'obturation (20) est réalisé en vue d'un verrouillage d'une ouverture de boîtier (42) délimitant le point de réception (44).

3. Appareil à main selon la revendication 1 ou 2, **caractérisé en ce que** le dispositif d'obturation (20) peut être amené automatiquement dans la position d'ouverture lors d'une activation de l'appareil.

4. Appareil à main selon l'une quelconque des revendications 1 à 3, **caractérisé en ce que** l'entraînement de bande (66) activé par un prébobinage de la bande de test (14) amène en même temps le dispositif d'obturation (20) dans la position d'ouverture.

5. Appareil à main selon l'une quelconque des revendications 1 à 4, **caractérisé en ce que** le dispositif d'obturation (20) peut être amené dans la position d'ouverture lors d'une prise en main manuelle de l'appareil et automatiquement dans la position de fermeture lorsqu'on lâche le boîtier (10).

6. Appareil à main selon l'une quelconque des revendications 1 à 5, **caractérisé en ce que** le dispositif d'obturation (20) présente un mécanisme à ressort (52) mise en tension dans la position d'ouverture de manière préférée par actionnement d'éléments de préhension (56) se trouvant au niveau du boîtier (10).

7. Appareil à main selon l'une quelconque des revendications 1 à 6, **caractérisé par** un élément de commutation destiné à une mise en marche automatique d'une alimentation électrique interne à l'appareil lors d'une ouverture du dispositif d'obturation (20).

8. Appareil à main selon l'une quelconque des revendications 1 à 7, **caractérisé en ce que** les sections de bande de test sont constituées de champs de réactif appliqués par sections sur une bande porteuse continue et réagissant à un composant du fluide corporel.

9. Appareil à main selon l'une quelconque des revendications 1 à 8, **caractérisé en ce que** l'unité de bande (12) et/ou l'unité de détection (16) peu(ven)t être déviée(s) par rapport au boîtier (10) à partir d'une position de travail en cas de choc contre un élément de rappel flexible (18).

10. Appareil à main selon la revendication 9, **caractérisé en ce que** l'unité de bande (12) et/ou l'unité de détection (16) peu(ven)t être déviée(s) vers l'intérieur du boîtier (10) à partir du point de réception (44).

11. Appareil à main selon la revendication 9 ou 10, **caractérisé en ce que** l'unité de bande (12) et l'unité de détection (16) peuvent sortir d'une position de butée mutuelle par l'intermédiaire d'un mécanisme de libération (30) agissant sur l'élément de rappel (18).

12. Appareil à main selon la revendication 11, **caractérisé en ce que** la tête de réception (40) constituée de manière préférée d'acier à ressort est réalisée de manière élastiquement déformable.
